# EUROPEAN PATENT SPECIFICATION

(11) **EP 2 213 276 B1**
(45) Date of publication and mention of the grant of the patent: **24.04.2013**
(21) Application number: 10151231.7
(22) Date of filing: 20.01.2010
(51) Int. Cl.: A61K 6/00, A61L 27/50

(54) **Multilayer device for forming adhesive pads for fixing dentures and for forming dermal adhesive bandages**
Mehrschichtige Vorrichtung zur Bildung von Klebepolstern zur Befestigung von Zahnprothesen und zur Bildung von dermalen Klebebinden
Dispositif multicouche pour la formation de plaques adhésives pour la fixation d'appareils dentaires et pour former des pansements adhésifs dermiques

(30) Priority: 29.01.2009 IT MI20090104
(43) Date of publication of application: 04.08.2010
(73) Proprietor: BIOFARMITALIA S.p.A., 20144 Milano (IT)
(72) Inventor: Brugali, Giuseppe, 24031, Almenno San Salvatore (BG) (IT); Pinna, Fausto, 20050, Lesmo (MI) (IT); Pinna, Marco, 21051, Arcisate (VA) (IT)
(74) Representative: Frignoli, Luigi

(56) References cited:
- EP-A2- 1 452 168
- WO-A2-2007/062346
- US-A- 4 632 880
- US-A1- 2005 137 272

## Description

The present invention relates to a multilayer device for forming adhesive pads for fixing dentures and dermal adhesive bandages.

Two types of formulation are used for fixing dentures into the oral cavity, namely a first type comprising mixtures of micro-crystalline waxes, modified celluloses, pectins, carrageenans and polybutane resins, and a second type comprising alginate threads and alginate particles combined with acrylic resins. In both cases the application presents a series of problems. With regard to products of the first type, these are difficult to use, as they have to be spread/secured on specific regions of the dental prosthesis, they do not provide a sufficient damping effect (elastic behaviour) to protect the palate or gum, and do not adequately conform to their morphological characteristics.

The products of the second type require, for developing a sufficient adhesiveness, a contact time with saliva liquids which is considerably longer than 60 seconds; it follows that fixing to the soft parts of the mouth is not adequate, resulting in denture displacement during mastication. This can also cause flaking of both types of product, with consequent release and possible ingestion of material particles.

US 2005/0137272 A1 describes gelled biopolymer based foam obtained by forming an aqueous dispersion sodium alginates (among other polymers), together with a gelling agent (comprising bivalent cations of alkaline materials, including calcium ions), a plasticizer (including glycerol), pH modifier and water forming a foam from the dispersion and forming a gelled foam, and then drying the gelled foam in a very time consuming manner (such as overnight, see examples 7, 17 and others). Such dried foam has high absorbency properties and its re-hydrating time is very long (such as 3-5 minutes, see example 13).

Such foam can be applied onto a surface of a single layer of a substrate (including a woven or non-woven article). This gelled form is obtained by heating to temperatures and for times variable between about 1 hour if at 80/100°C and about 8 hours if at ambient temperature. It follows that the residual water content of the dried gelled foam at ambient humidity is about 10% (see examples 19, 20 and elsewhere). Such a low residual water content of the dried product has the drawback that the product (foam) is too rigid and poorly manageable for its use for fixing dentures in the oral cavity. Moreover, applying the foam to just a single layer of a substrate easily leads to detachment of this foam from the support. The aforesaid US patent states that the dried foam is able to absorb large quantities of aqueous liquids, for which reason it illustrates its use for forming bandages which can be applied directly to wounds, to hence absorb the exudates and become adhesive to the skin by the effect of the various bivalent cations present in said exudates.

The main object of the present invention is to provide a device able to fix dentures into the oral cavity and fix bandages onto the human skin without presenting the aforesaid drawbacks, i.e. a device which is soft, flexible and permeable to aqueous liquids, hence usable both for applying and retaining dentures within the oral cavity and for forming dermal adhesive bandages.

This object is attained by a device characterised by consisting of two outer layers and an intermediate layer in the form of an aqueous gel having a calcium ion and/or magnesium ion content less than 0.04%, a sodium alginate content between 18.0% and 22.0%, a glycerol content between 30.0% and 42.0%, and a water content between 30.0% and 50.0%, the percentages being by weight on the weight of the intermediate layer.
Preferably the sodium alginate content of the device is 20%, the glycerol content is 40%, and the constituent material of the outer layers is a web made of hydrophilic fibres, and can comprise a substance of cosmetic or therapeutic action, in particular β-cyclodextrin.

The multilayer device of the present invention is prepared by a process **characterised in that** between 9.0% and 11.0% of sodium alginate and between 15.0% and 21.0% of glycerol are mixed with an aqueous liquid having a calcium ion and/or magnesium ion content less than 0.02%, the percentages indicated being by weight on the total weight of the mixture, which is spread and laminated between two layers of soft, flexible material permeable to aqueous liquids, in a quantity between 400 and 420 g/m² such as to form between them said intermediate layer forming part of a multilayer device which is partially dried at a temperature between 100°C and 110°C for a time between 3 and 5 minutes (hence for a very short time compared with that suggested by the aforesaid US patent).

The advantages deriving therefrom are that the two web layers between which the intermediate layer is interposed prevent its flaking, that the thickness of said intermediate layer can be easily obtained and gauged by common laminating machines, that the absorption of water (containing calcium and/or magnesium cations with consequent exhibition of the adhesive power) for gel formation takes place in a very short time, of just a few seconds (generally less than 10 seconds).

The rate of re-hydration of the previously dried device is high because the thickness which has to be traversed from the outer surfaces of the two web layers to the intermediate layer is small, the residual water content of the dried product developing the property of conferring softness on the multilayer device.

US 4632880 describes an adhesive device formed from heat-laminated webs having a water activated adhesive uniformly dispersed between these webs, to support a denture within the oral cavity. However, this device comprises - as an essential component bonding the fibres together- a binder based on polyethylene oxide polymer together with sodium alginate as a water-activated adhesive.

EP1452168 describes a pad comprising a flexible support comprising various components spread over one surface thereof, these components having as is basic constituent a product inherently adhesive towards the skin, in particular polyvinyl alcohol.

Evidently, the adhesive power of the device of the present invention is exhibited if, and only if, there is an abundant availability of calcium and/or magnesium cations in the liquid (human saliva, wound exudates, artificial aqueous solutions or common tap water). The calcium and/or magnesium cations must however be absent (or present only in the minimum quantities specified in claim 7) during the device coupling/drying stage (production stage) due to the irreversibility of the gel formation process, which would make it difficult to spread the gel over the surfaces of the two webs.
For a better understanding of the characteristics of the device of the present invention, two non-limiting embodiments will now be described.

### EXAMPLE 1

### PREPARATION OF AN ADHESIVE PAD FOR FIXING DENTURES WITHIN THE ORAL CAVITY

Sodium alginate is dispersed in water (with the absence of calcium or magnesium cations or containing less than 0.02% of these cations) in a stainless steel vessel. Mixing must continue until the polymer powder is completely dissolved (requiring about 45-50 minutes). On termination of this step, a very viscous transparent liquid mass forms. Glycerol (pharmaceutical grade) is then added into the same vessel, and left under agitation for a further 15 minutes.

At the end of this step an aqueous solution containing 10 wt% of sodium alginate and 20 wt% of glycerol is obtained. The solution, which is colourless and tasteless, if not used immediately can be stored in a refrigerator cell at +4°C for not more than 48 hours (in closed steel vessels).

The solution is then applied by lamination between two layers of non-woven hydrophilic web in a quantity of 420 g/m².

The coupled system formed in this manner is maintained extended and is advanced along a tunnel drying oven of hot air jet type. Its four successive sections must have the following temperatures: a) +100°C, b) +100°C, c) +100°C, and d) +110°C, its advancement rate being 3.5 m/sec. The total residence time is 5 min, equally divided between the various sections.

The product obtained is wound into rolls and then divided into smaller rolls to be fed to subsequent punching to obtain the required shapes.

The shaped pads obtained are then inserted into individual sachets, which are then inserted into boxes for final packaging.
Immediately prior to use the pads are preferably re-hydrated with potable water (containing calcium and/or magnesium cations) to enable rapid commencement of gel swelling by re-hydration followed by easy manipulation in applying the pad to the denture and into the oral cavity, where the presence of the bivalent cations in the water and saliva increases the adhesivity of the device both to the denture and to the oral cavity mucosa.

### EXAMPLE 2

### Preparation of a dermal adhesive bandage for thalassotherapeutic treatment of the skin

Sodium alginate is dispersed in pure water (i.e. without calcium and/or magnesium cations) under slow stirring and at ambient temperature in a stainless steel vessel (easily washable and sanitizable). Mixing must continue until the polymer powder is completely dissolved (requiring about 60 minutes). On termination of this step, a very viscous transparent liquid mass forms. Glycerol (pharmaceutical grade) is then added in the same vessel, and left under agitation for a further 15 minutes.

At the end of this step an aqueous solution containing 11 wt% of sodium alginate and 15 wt% of glycerol is obtained.

Simultaneously, in another vessel, an aqueous solution of the following salts is prepared (percentages by weight on the total solution weight):
1) magnesium chloride MgCl₂ 0.08%
2) potassium sulphate K₂SO₄ 1.4%
3) sodium chloride NaCl 10.0%
4) zinc sulphate ZnSO₄ 0.4%
5) sodium bicarbonate NaHCO₃ 0.04%
6) potassium bromide KBr 0.02%

This saline solution is qualitatively similar to that of the Dead Sea.

At this point 50% w/w of the sodium alginate solution is mixed with 50% w/w of the saline solution in a third mixer under mild stirring.
The mass is kept under agitation until completely homogenized (about 30 minutes).

The weight percentage concentration of the solutes is hence halved compared with that specified in the starting solutions:
1) sodium alginate 5.5%
2) glycerol 7.5%
3) magnesium chloride MgCl₂ 0.04%
4) potassium sulphate K₂SO₄ 0.07%
5) sodium chloride NaCl 5.0%
6) zinc sulphate ZnSO₄ 0.2%
7) sodium bicarbonate NaHCO₃ 0.02%
8) potassium bromide KBr 0.01 %

In this manner a gel is obtained (certainly partially crosslinked because of the presence of the magnesium cation), and is laminated by application onto the opposing surfaces of two layers of non-woven web, the gel quantity being 400 g/m² of web.

The coupled system formed in this manner is maintained extended and is advanced through a tunnel drying oven of hot air jet type. Its four progressive sections must have the following temperatures: a) +100°C, b) +100°C, c) +100°C, and d) +110°C, its advancement rate being 3.5 m/sec, the total residence time in the oven being 4 min, equally divided between the various oven sections.

The final, i.e. coupled, product is wound into rolls and then divided into smaller rolls to be fed to subsequent punching to obtain the required shapes.

The shaped sheets obtained are then inserted into sachets, which are then inserted into boxes for final packaging.

The result obtained is a rectangular sheet which can be further cut to size by the final user to better adapt it to the body part to be treated.

Application requires prior wetting of the sheets with warm potable water (containing calcium and magnesium cations) and their application to the skin by handling them as though they were bandages. The water absorbed by capillarity by the two outer non-woven fabric layers comes into contact with the intermediate layer which, as it contains sodium alginate fibres, begins to swell (increasing in volume). The presence of the calcium and/or magnesium cations originating from the hardening salts present in the potable water completes gelling of the active mass by forming ionic cross-linkages between the alginate polysaccharide chains (gel cross-linkage).

The bandage obtained in this manner is suitable for application to any part of the body except injured parts.

The high local concentration of saline solutes remains substantially constant for the entire treatment period, enabling maximum therapeutic effectiveness of the saline complex (there is however some water loss by evaporation due to body heat).

This device can be produced by preparing the starting gel directly from thermal waters to hence obtain therapeutic bandages with characteristics depending on those of the thermal water used.

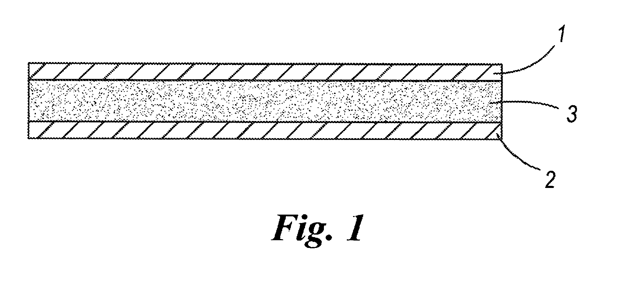

A cross-section through a bandage is shown on an enlarged scale in the accompanying Figure 1 in which the reference numerals 1 and 2 indicate respectively two separate layers of hydrophilic porous web fabric between which the intermediate dried gel layer 3 is interposed.

## Claims

1. A soft, flexible multilayer device permeable to aqueous liquids for forming adhesive pads for fixing dentures and for forming dermal adhesive bandages, **characterised by** consisting of two outer layers and an intermediate layer in the form of an aqueous gel having a calcium ion and/or magnesium ion content less than 0.04%, a sodium alginate content between 18.0% and 22.0%, a glycerol content between 30.0% and 42.0%, and a water content between 30.0% and 50.0%, the percentages being by weight on the weight of the intermediate layer.

2. A device as claimed in claim 1, **characterised in that** the sodium alginate content is 20%.

3. A device as claimed in claims 1 and 2, **characterised in that** said glycerol is present in a quantity of 40.0%.

4. A device as claimed in claim 1, **characterised in that** said outer layers of soft material consist of webs formed of hydrophilic fibres.

5. A device as claimed in claim 1, **characterised by** comprising at least one substance of cosmetic or therapeutic action.

6. A device as claimed in claim 5, **characterised in that** said substance is β-cyclodextrin.

7. A process for preparing a multilayer device in accordance with claims from 1 to 6, **characterised in that** between 9.0% and 11.0% of sodium alginate and between 15.0% and 21.0% of glycerol are mixed with an aqueous liquid having a calcium ion and/or magnesium ion content less than 0.02%, the percentages indicated being by weight on the total weight of the mixture, which is spread and laminated between two layers of soft, flexible material permeable to aqueous liquids, in a quantity between 400 and 420 g/m². such as to form between them said intermediate layer forming part of a multilayer device which is partially dried at a temperature between 100°C and 110°C for a time between 3 and 5 minutes.

## Patentansprüche

1. Weiche, flexible mehrschichtige Vorrichtung, die für wässrige Flüssigkeiten durchlässig ist, für die Bildung von Haftpads zum Fixieren von Zahnersatz und für die Bildung von dermalen Haftverbänden, **dadurch gekennzeichnet, dass** sie aus zwei äußeren Schichten und einer Zwischenschicht in der Form eines wässrigen Gels, das einen Calciumionen- und/oder Magnesiumionengehalt kleiner als 0,04%, einen Natriumalginatgehalt zwischen 18,0% und 22,0%, einen Glyceringehalt zwischen 30,0% und 42,0% und einen Wassergehalt zwischen 30,0% und 50,0% hat, wobei die Prozentwerte in Gewicht zu dem Gewicht der Zwischenschicht sind, besteht.

2. Vorrichtung wie in Anspruch 1 beansprucht, **dadurch gekennzeichnet, dass** der Natriumalginatgehalt 20% ist.

3. Vorrichtung wie in den Ansprüchen 1 und 2 beansprucht, **dadurch gekennzeichnet, dass** das genannte Glycerin in einer Menge von 40,0% vorliegt.

4. Vorrichtung wie in Anspruch 1 beansprucht, **dadurch gekennzeichnet, dass** die genannten äußeren Schichten aus weichem Material aus Geweben, die aus hydrophilen Fasern gebildet sind, bestehen.

5. Vorrichtung wie in Anspruch 1 beansprucht, **dadurch gekennzeichnet, dass** sie mindestens eine Substanz mit kosmetischer oder therapeutischer Wirkung umfasst.

6. Vorrichtung wie in Anspruch 5 beansprucht, **dadurch gekennzeichnet, dass** die genannte Substanz β-Cyclodextrin ist.

7. Verfahren zur Herstellung einer mehrschichtigen Vorrichtung gemäß den Ansprüchen 1 bis 6, **dadurch gekennzeichnet, dass** zwischen 9,0% und 11,0% Natriumalginat und zwischen 15,0% und 21,0% Glycerin mit einer wässrigen Flüssigkeit, die einen Calciumionen- und/oder Magnesiumionengehalt kleiner als 0,02% hat, gemischt werden, wobei die Prozentwerte angegeben sind als Gewicht zu dem Gesamtgewicht der Mischung, die in einer Menge zwischen 400 und 420 g/m² zwischen zwei Schichten aus weichem, flexiblem Material, das durchlässig für wässrige Flüssigkeiten ist, aufgetragen und laminiert wird, um zwischen ihnen die genannte Zwischenschicht, die einen Teil einer mehrschichtigen Vorrichtung bildet, zu bilden, die bei einer Temperatur zwischen 100°C und 110°C für einen Zeitraum zwischen 3 und 5 Minuten teilweise getrocknet wird.

## Revendications

1. Dispositif multicouche flexible et mou perméable aux liquides aqueux pour former des plaques adhésives pour fixer des appareils dentaires et pour former des pansements adhésifs dermiques, **caractérisé en ce qu'**il est constitué de deux couches externes et d'une couche intermédiaire sous la forme d'un gel aqueux présentant une teneur en ion calcium et/ou en ion magnésium inférieure à 0,04 %, une teneur en alginate de sodium comprise entre 18,0 % et 22,0 %, une teneur en glycérol comprise entre 30,0 % et 42,0 %, et une teneur en eau comprise entre 30,0 % et 50,0 %, les pourcentages étant exprimés en poids par rapport au poids de la couche intermédiaire.

2. Dispositif selon la revendication 1, **caractérisé en ce que** la teneur en alginate de sodium est de 20 %.

3. Dispositif selon les revendications 1 et 2, **caractérisé en ce que** ledit glycérol est présent en une quantité de 40,0 %.

4. Dispositif selon la revendication 1, **caractérisé en ce que** lesdites couches externes de matériau mou sont constitués de toiles formées de fibres hydrophiles.

5. Dispositif selon la revendication 1, **caractérisé en ce qu'**il comprend au moins une substance ayant une action cosmétique ou thérapeutique.

6. Dispositif selon la revendication 5, **caractérisé en ce que** ladite substance est de la β-cyclodextrine.

7. Procédé de préparation d'un dispositif multicouche selon les revendications 1 à 6, **caractérisé en ce qu'**entre 9,0 % et 11,0 % d'alginate de sodium et entre 15,0 % et 21,0 % de glycérol sont mélangés avec un liquide aqueux présentant une teneur en ion calcium et/ou en ion magnésium inférieure à 0,02 %, les pourcentages indiqués étant exprimés en poids par rapport au poids total du mélange, qui est étalé et stratifié entre deux couches de matériau flexible mou perméable aux liquides aqueux, en une quantité comprise entre 400 et 420 g/m², de façon à former entre elles ladite couche intermédiaire faisant partie d'un dispositif multicouche qui est partiellement séché à une température comprise entre 100 °C et 110 °pendant une durée comprise entre 3 et 5 minutes.
